Europäisches Patentamt

European Patent Office

Office européen des brevets

(19)

(11) Numéro de publication: **0 175 618**
**B1**

(12) # FASCICULE DE BREVET EUROPÉEN

(45) Date de publication du fascicule du brevet:
21.06.89

(51) Int. Cl.⁴: **G 01 N 33/49**

(21) Numéro de dépôt: **85401778.7**

(22) Date de dépôt: **13.09.85**

(54) Dispositif de séparation du plasma des autres constituants d'un liquide sanguin.

(30) Priorité: **18.09.84 FR 8414272**

(43) Date de publication de la demande:
**26.03.86 Bulletin 86/13**

(45) Mention de la délivrance du brevet:
**21.06.89 Bulletin 89/25**

(84) Etats contractants désignés:
**BE DE GB IT**

(56) Documents cité:
**US-A-4 354 116**

(73) Titulaire: **CENTRE REGIONAL DE TRANSFUSION SANGUINE, 1 Bld Alexandre Fleming BP 1937, F-25020 Besancon (FR)**

(72) Inventeur: **Masse, Maurice, Chemaudin, F-25320 Montferrand le Chateau (FR)**
Inventeur: **Carquille, Bernard, F-25870 Geneuille (FR)**
Inventeur: **Guignard, Michel, Les Varennes Auxon Dessus, F-25870 Geneuille (FR)**

(74) Mandataire: **Derambure, Christian, BUGNION ASSOCIES 55, rue Boissonade, F-75014 Paris (FR)**

## Description

L'invention concerne un dispositif de séparation des plaquettes et des globules rouges du sang, afin d'obtenir le plasma seul.

On connaît des dispositifs et des procédés permettant de séparer les différents constituants du sang. Un procédé couramment employé est de prélever le sang, notamment le sang humain, dans des ensembles stériles de trois poches réunies par des conduits. L'ensemble est souple et transparent. La première poche (M) est destinée à récolter le sang prélevé, la première poche satellite (S) est destinée à recueillir le plasma après centrifugation, la deuxième poche satellite (A) est destinée à contenir d'autres constituants, tandis que la poche initiale (M) est destinée à contenir les globules rouges après traitement.

Après prélèvement, on effectue une centrifugation des trois poches ensemble. Puis on introduit la poche (M) dans un coin. Ce coin est constitué dans la technique actuelle par un premier mors vertical, fixe et un second mors incliné par rapport au premier, mobile et rappelé vers le mors fixe par un ressort travaillant en contraction, qui lorsqu'il n'est pas soumis à une force écartant le mors mobile du mors fixe, fait pivoter le mors incliné mobile autour d'un axe horizontal, disposé à la base du mors vertical fixe. La poche contient le sang centrifugé et donc séparé en trois couches, la couche inférieure contenant les globules rouges, la couche intermédiaire contenant les globules blancs et les plaquettes, et la couche supérieure étant constituée de plasma. La poche est comprimée par le mors mobile qui pivote par rapport au mors fixe. Comme la poche (M) comporte un conduit, le liquide de la couche supérieure s'écoule par le conduit et le plasma s'écoule dans la première poche satellite (5). Ainsi le plasma est séparé. Le document US-A-4 354 116 décrit un tel dispositif.

Cependant, un tel procédé de séparation de plasma utilisé couramment dans la technique antérieure ne permet pas d'obtenir un plasma très pur et exempt de globules blancs et de plaquettes. En effet, l'instant où l'écoulement du plasma dans le conduit est arrêté est choisi par un ou une opératrice qui, à l'oeil nu, décide, en fonction de la couleur de l'interface de plasma/couche intermédiaire d'arrêter l'écoulement du plasma. De plus, une telle opération manuelle est longue et d'autre part il est souhaitable d'automatiser la séparation du plasma des globules blancs et des globules rouges car il est nécessaire de travailler en milieu complètement stérile ou à tout le moins en milieu non contaminant.

Par conséquent, l'invention vise à pallier ces inconvénients

Un but de l'invention est de fournir un dispositif de séparation du plasma des globules blancs et des globules rouges du sang, la séparation étant automatique et le plasma obtenu étant exempt de globules blancs et de plaquettes.

A cet effet, l'invention concerne un dispositif de séparation des globules rouges du plasma sanguin constitué d'un conteneur souple, transparent notamment à la lumière artificielle, pour liquide sanguin ayant préalablement subi une centrifugation, une tubulure permettant l'évacuation du contenu de la poche, un mors fixe plan, vertical et un mors plan, incliné, mobile à pivotement autour d'un axe de rotation horizontal, disposé à la base du mors fixe, le mors fixe et le mors mobile étant solidarisés par des moyens élastiques rappelant le mors mobile vers le mors fixe, des moyens de maintien du conteneur en position verticale et fixe par rapport au mors vertical, des moyens d'émission et de réception d'un faisceau lumineux traversant les parois du conteneur, le liquide sanguin, et utilisés pour détecter la séparation entre les globules blancs et les globules rouges.

Un tel dispositif selon l'invention est caractérisé en ce qu'il comporte deux bras portant respectivement les moyens d'émission et les moyens de réception du faisceaux lumineux, ces bras étant maintenus parallèles lors de la séparation du plasma et temporairement dissociables de manière à permettre la mise en plan ou l'extraction du conteneur sur les moyens de maintien.

L'invention se caractérise en outre par le fait que la référence utilisée comme couleur de référence du plasma est une référence mobile, à savoir pour chaque mesure la référence est constituée par la couleur du plasma seul et on mesure la couleur du liquide sanguin se trouvant dans le conteneur par rapport à la couleur du plasma pur.

Enfin le dispositif selon la présente invention comporte des moyens de mesure de l'intensité lumineuse transmise et des moyens ayant pour fonction de comparer la valeur de l'intensité lumineuse transmise à un instant t1 par rapport à la valeur de l'intensité lumineuse transmise à un instant t0, lors de la transmission à travers le plasma pur exempt de globules rouges, de globules blancs et de plaquettes, et ce pour chaque séparation effectuée. Il comporte des moyens d'émission d'un signal électrique dont l'intensité est fonction de l'intensité lumineuse transmise, des moyens de fermeture d'un circuit d'écoulement du plasma hors du conteneur, actifs lorsque le signal électrique correspond à une intensité lumineuse transmise qui est différente de l'intensité lumineuse de référence.

La description suivante, en regard des dessins annexés à titre d'exemple non limitatif permettra de comprendre comment l'invention peut être mise en pratique.

La figure 1 est une vue de profil du dispositif selon l'invention.

La figure 2 est une vue de côté des mors du dispositif munis des moyens d'émission et de réception du faisceau lumineux, notamment.

La figure 3 est une vue de face de l'objet de la figure 2.

La figure 4 est une vue schématique d'une courbe d'enregistrement du signal électrique correspondant au faisceau lumineux transmis.

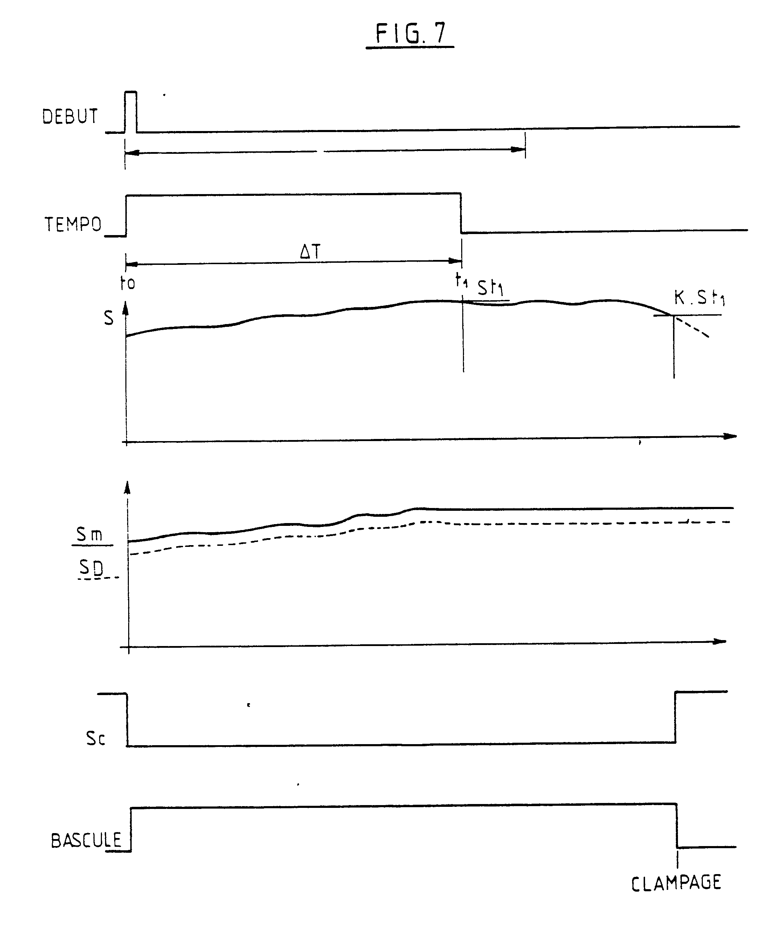

La figure 5 est un schéma du conteneur disposé entre les deux mors.

La figure 6 est un schéma montrant les moyens mis en oeuvre pour l'enregistrement des signaux électriques et la commande de la fermeture du conduit de sortie du plasma.

La figure 7 et un schéma du diagramme temporel du dispositif selon l'invention.

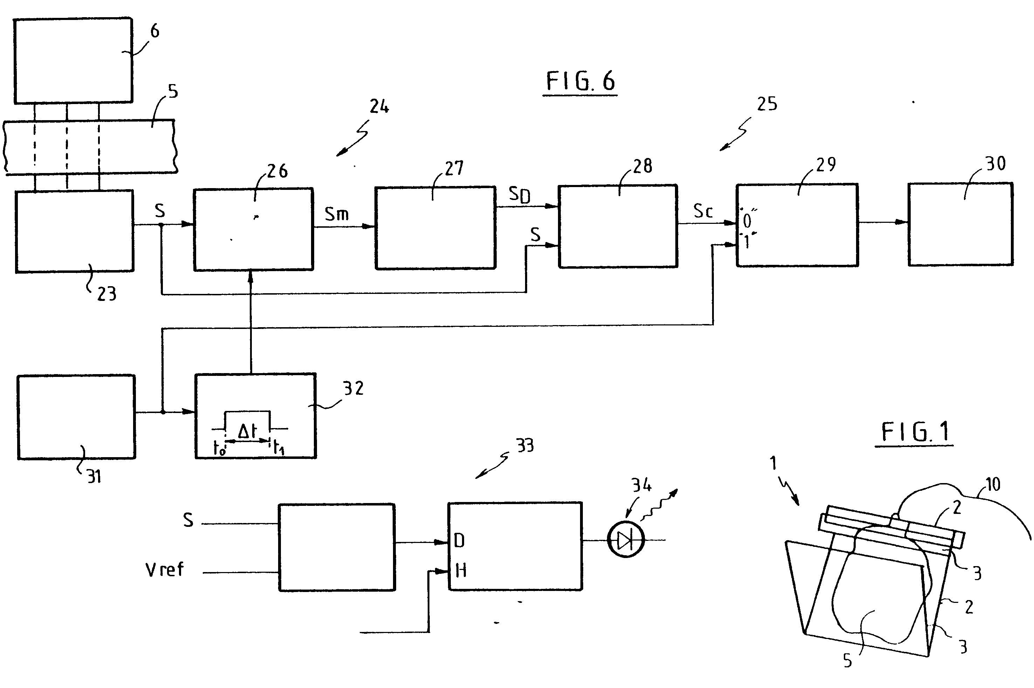

Le dispositif de séparation 1 représenté plus particulièrement sur la figure 1 est constitué d'un premier mors 2 qui est plan et pseudo rectangulaire. Ce mors 2 est vertical et fixe. Un second mors 3 est plan et incliné par rapport au premier mors 2. Ce mors 3 est lui aussi pseudo rectangulaire. Il est mobile à pivotement autour d'un axe XX de rotation. Cet axe XX est horizontal et disposé à la partie extrême inférieure 4 du mors fixe 2.

Le dispositif représenté sur la figure 1 est montré à l'état initial, à savoir avant que le mors mobile 3 se soit rabattu sur le mors fixe 2. Entre les deux mors 2 et 3 on a placé une poche transparente 5. Cette poche 5 est transparente au rayonnement lumineux envoyé par les moyens d'émission 6 d'un faisceau lumineux 7.

La poche 5 contient un liquide sanguin 8 qui a subi préalablement une centrifugation. Ce sang est alors constitué de trois couches superposées. La couche inférieure contient les hématies ou globules rouges, la couche intermédiaire contient les globules blancs et les plaquettes, tandis que la couche supérieure ne contient que du plasma. Cependant, le plasma de la couche supérieure est pur au niveau le plus haut, mais au fur et à mesure que l'on se rapproche de la couche contenant les plaquettes et les globules blancs, le plasma est contaminé par ceux-ci et il existe un gradient de concentration en plaquettes croissant depuis le niveau supérieur de la couche supérieure vers la couche intermédiaire. Il s'agit donc d'obtenir un plasma ne contenant qu'un nombre faible de plaquettes résiduelles.

Le mors mobile 3 est rappelé vers le mors fixe 2 par un ressort (non représenté) qui a une extrémité fixée sur la partie inférieure du mors fixe 2 et son autre extrémité fixée sur le mors mobile 3. D'une manière connue la poche 5 comporte à sa partie supérieure 9 un orifice de sortie qui est relié à une tubulure 10 qui permet au plasma d'être évacué de la poche 5, lorsque la poche 5 est comprimée par le mors mobile 3. En effet la poche 5 est placée entre le mors mobile 3 et le mors fixe 2.

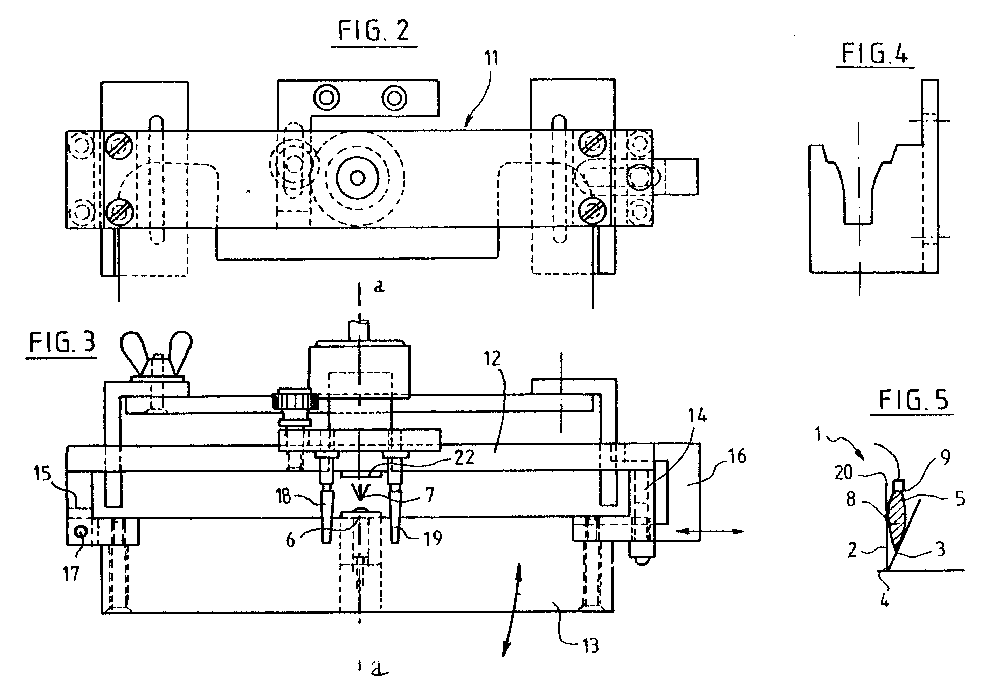

La poche 5 est maintenue entre les deux mors 2 et 3 par des moyens de maintien 11. Ces moyens de maintien 11 sont constitués selon l'invention par un étrier comportant deux bras 12 et 14 (voir figures 2 et 3) mobiles l'un par rapport à l'autre lors de la mise en place de la poche 5. De préférence, le bras 13 est en un matériau transparent aux rayons lumineux utilisés pour effectuer la séparation du plasma. Plus préférentiellement, le bras 13 est en "PLEXIGLASS".

Les deux bras 12 et 13 sont maintenus parallèles lors de la séparation du plasma. Le parallèlisme est obtenu par des entretoises 14 et 15 vissées sur le bras 12. La rotation du bras 13 par rapport au bras 12 est obtenue par un verrou 16 à mouvement latéral qui libère le bras 13 par rapport au bras 12 et permet la rotation autour de l'axe 17. La poche 5 est positionnée entre les deux bras 12 et 13 grâce à deux plots 18, 19 qui correspondent à deux trous (non représentés) effectués dans la partie extrême supérieure 20 de la poche 5. Cela permet à la poche 5 d'être suspendue et de conserver une position fixe par sport aux bras 12 et 13.

Selon l'invention, le dispositif comporte des moyens d'émission 6 d'un faisceau lumineux, ces moyens d'émission étant fixes par rapport au conteneur ou plus particulièrement par rapport à la poche 5. En effet, les moyens d'émission 6 sont fixés sur le bras 13. Plus particulièrement, les moyens d'émission sont fixes par rapport à la paroi mobile externe 21 située en regard du mors mobile 3.

Le bras 13 supporte les moyens d'émission 6 du faisceau lumineux tandis que le bras 12 supporte les moyens de réception 22 du faisceau lumineux transmis après que celui-ci ait traversé les parois transparentes de la poche 5 et du liquide sanguin.

L'axe optique aa entre la cellule émettrice 6 et la cellule de détection 22 est donc solidaire de la poche 5 contenant le sang mais est mobile par rapport au coin formé par le mors fixe 2 et le mors mobile 3.

Selon l'invention, la séparation du plasma est donc faite en fonction de la lumière transmise par le plasma, les cellules émettrices 6 et réceptrices 22 étant fixes par rapport au conteneur 5.

Le dispositif selon l'invention comporte en outre des moyens d'émission 23 d'un signal électrique en fonction de l'intensité lumineuse transmise et des moyens de mesure 24 ayant pour fonction de mesurer la valeur de l'intensité lumineuse transmise à un instant t1 par rapport à la valeur de l'intensité lumineuse transmise à l'instant t0, lors de la transmission à travers le plasma pur exempt de plaquettes et de globules blancs, et ce pour chaque liquide sanguin différent.

Ainsi, pour chaque liquide sanguin différent, le dispositif mesure l'intensité lumineuse transmise après passage du faisceau lumineux au travers du plasma, à un niveau donné, par rapport à l'intensité lumineuse transmise après passage du faisceau lumineux à travers le plasma pratiquement pur, donc à travers une couche supérieure du plasma. Ainsi, selon l'invention, on utilise une valeur de référence mobile qui est fonction de chaque plasma pour chaque liquide sanguin différent.

Lorsque l'intensité lumineuse transmise à une valeur donnée différente de l'intensité lumineuse transmise de référence, c'est-à-dire lorsque le signal électrique correspondant à la valeur de l'intensité lumineuse transmise, a une valeur donnée différente du signal électrique de référence, on désire fermer le conduit d'écoulement ou tubulure 10. A cet effet, le dispositif comporte des moyens de fermeture de la tubulure 10. Ces moyens peuvent être automatiques ou manuels. En tous cas, selon l'invention, les moyens de fermeture sont commandés par des moyens de commande électroniques 25. Ces moyens électroniques comprennent: une mémoire 26, suivie d'un diviseur de tension 27, un comparateur de tension 28, une bascule 29 et un circuit de puissance 30. En parallèle se trouvent des moyens d'émission d'un signal 31 correspondant au début de l'extraction ou écoulement du plasma, et des moyens de temporisation 32. Le dispositif comporte en outre un circuit de détection automatique de rejet 33 des plasmas impropres à l'utilisation. En effet, des plasmas peuvent contenir trop de graisses et ne peuvent donc pas être utilisés directement en perfusion. Il est donc nécessaire de les repérer automatiquement. Ainsi, le dispositif comporte en outre un circuit annexe comportant un comparateur 34 et une bascule 35.

Lorsque la poche 5 est placée entre les mors fixe 2 et mobile 3, un déplacement mécanique correspondant à la fin de l'opération de chargement provoque l'apparition d'une impulsion électrique qui déclenche le circuit de temporisation et met à l'état 1 logique la bascule 29.

Jusqu'à l'instant t1 qui correspond à la fin de la temporisation, le signal Sm qui sort de la mémoire analogique suit le signal de mesure de la lumière S. On à donc Sd = K.Sm.

K est une constante inférieure à 1, par exemple 0,9 ce qui entraîne $\underline{Sd}$/s. A la sortie du comparateur 28 on a donc Sc = O logique.

Après une durée de temps t1, Sm garde la valeur St1 de S à l'instant t1. S évolue peu et les équations précédentes sont encore exactes.

A l'apparition du plasma comportant un nombre important de plaquettes et globules blancs, le signal S diminue rapidement jusqu'à S/Sd, ce qui modifie la sortie du comparateur et Sc = 1 logique.

La bascule passe à l'état 0, commandant le clampage de la tubulure 10 ou tube d'écoulement du plasma, par l'intermédiaire du circuit de puissance 30.

Pour le rejet des plasmas gras, le signal S est comparé à une tension de référence Vref. Le résultat de cette comparaison est recopié par une bascule 33 lors de l'impulsion donnée au début de la séparation. La sortie de la bascule peut commander l'allumage d'une lampe 34 ou tout autre dispositif de puissance adéquat.

Selon l'invention on prévoit un ensemble qui comporte une pluralité de dispositifs de détection, chacun étant relié à une poche, et comportant des moyens de contrôle simultané d'une pluralité de circuits de détection 33. Ainsi, par un simple développement des circuits électroniques, cet ensemble a la possibilité de contrôler simultanément plusieurs détections.

## Revendications

1. Dispositif de séparation des globules rouges du plasma sanguin constitué d'un conteneur souple (5), transparent notamment à la lumière artificielle, pour liquide sanguin ayant préalablement subi une centrifugation, une tubulure (10) permettant l'évacuation du contenu de la poche (5), un mors fixe (2) plan, vertical et un mors plan, incliné, mobile (3) à pivotement autour d'un axe de rotation horizontal, disposé à la base du mors fixe (2), le mors fixe (2) et le mors mobile (3) étant solidarisés par des moyens élastiques rappelant le mors mobile (3) vers le mors fixe (2), des moyens de maintien (11) du conteneur (5) en position verticale et fixe par rapport au mors vertical (2), des moyens d'émission (6) et de réception (22) d'un faisceau lumineux traversant les parois (21, 21') du conteneur (5), le liquide sanguin, et utilisés pour détecter la séparation entre les globules blancs et les globules rouges caractérisé en ce qu'il comporte deux bras (12 et 13) portant respectivement les moyens d'émission (6) et les moyens de réception (22) du faisceaux lumineux, ces bras étant maintenus parallèles lors de la séparation du plasma et temporairement dissociables de manière à permettre la mise en plan ou l'extraction du conteneur sur les moyens de maintien (11).

2. Dispositif de séparation selon la revendication 1, caractérisé en ce qu'il comporte des entretoises (14, 15) maintenant le parallélisme des bras lors de la séparation du plasma.

3. Dispositif de séparation selon la revendication 2, caractérisé en ce que les bras 12 et 13 sont articulés l'un par rapport à l'autre autour d'un axe 17 et qu'il comporte un verrou 16 susceptible de bloquer cette articulation lors de la séparation du plasma et de le libérer pour permettre la mise en place du conteneur sur les moyens de maintien (11).

4. Dispositif de séparation selon l'une quelconque des revendication 1 à 3, caractérisé par le fait qu'il comporte des moyens d'émission d'un signal électrique (23) en fonction de l'intensité lumineuse transmise et des moyens ayant pour fonction de mesurer la valeur de l'intensité lumineuse transmise à un instant t1 par rapport à la valeur de l'intensité lumineuse de référence transmise à un instant t0, lors de la transmission à travers le plasma pur exempt de plaquettes et de globules blancs, et ce pour chaque liquide sanguin différent, des moyens de fermeture (25) d'un circuit d'écoulement du plasma hors du conteneur (5), actifs lorsque le

signal électrique (23) correspond à une intensité lumineuse transmise à une valeur donnée par rapport à l'intensité lumineuse de référence et agissent sur la tubulure (10).

5. Dispositif de séparation selon la revendication 4, caractérisé par le fait qu'il comporte des moyens (25) de fermeture de la tubulure (10) comprenant une mémoire (26), un diviseur de tension (27), un comparateur de tension (28), une bascule (29) et un circuit de puissance (30) placés en série et des moyens d'émission (31) d'un signal correspondant au début de l'écoulement du plasma, et des moyens de temporisation (32), placés en parallèle.

6. Dispositif de détection selon la revendication (5) caractérisé en ce qu'il comporte un circuit de détection automatique (33) de rejet des plasmas impropres à l'utilisation, constitué d'un circuit annexe comportant un comparateur (34) et une bascule (35).

**Patentansprüche**

1. Vorrichtung zur Trennung der roten Blutkörperchen im Blutplasma, bestehend aus einem, insbesondere im künstlichen Licht transparenten, flexiblen Behälter (5) für vorher zentrifugierte Blutflüssigkeit, einem Stutzen (10) zum Ablaß des Inhalts des Beutels (5), einer festen, flachen, vertikalen Spannbacke (2) sowie einer flachen, schrägen, beweglichen Spannbacke (3), die um eine an der Basis der festen Spannbacke (2) befindliche horizontale Drehachse geschwenkt werden kann, wobei die feste Spannbacke (2) und die bewegliche Spannbacke (3) mit elastischen Vorrichtungen miteinander verbunden sind und dei Bewegliche Spannbacke (3) von der festen Spannbacke (2) zurückgerufen wird; des weiteren Vorrichtungen (11) zum Halt des Behälters (5) in vertikaler und gefestigter Position in bezug auf die vertikale Spannbacke (2) sowie Vorrichtungen zur Aussendung (6) und zum Empfang (22) eines die Wände (21, 21') des Behälters (5) und die Blutflüssigkeit durchqerenden Lichtsstrahls, die zur Erfassung der erfolgten Trennung zwischen weißen und roten Blutkörperchen dienen. Die Vorrichtung ist dadurch gekennzeichnet, daß sie über zwei Arme (12 und 13) verfügt, die jeweils mit Vorrichtungen zur Aussendung (6) und zum Empfang (22) des Lichtstrahls versehen sind, wobei die Arme bei der Plasmatrennung parallel angebracht sind und verübergehend voneinander getrennt werden können, um das Einsetzten des Behälters in oder das Entfernen desselben aus der Haltvorrichtung (11) zu ermöglichen.

2. Trennvorrichtung nach Anspruch 1, dadurch gekennzeichnet, daß sie mit Stegen (14, 15) zur Sicherstellung der Arm-Parallelität bei der Plasmatrennung versehen ist.

3. Trennvorrichtung nach Anspruch 2, dadurch gekennzeichnet, daß die Arme 12 und 13 beweglich zueinander angeordnet und um die Achse 17 schwenkbar sind, wobei die Vorrichtung mit einem Verriegelungssystem 16 versehen ist, das zur Plasmatrennung das Gelenk blockiert und es zur Anbringung des Behälters an den Haltvorrichtungen (11) weider entriegelt.

4. Trennvorrichtun nach einem der Ansprüche 1 bis 3, dadurch gekennzeichnet, daß sie mit Vorrichtungen zur Aussendung eines von der Stärke des übertragenen Lichts abhängigen elektrischen Signals (23) versehen ist, sowie mit Vorrichtungen, die zur Messung der zum Zeitpunkt t1 übertragenen Lichstärke dienen, und zwar in Abhängigkeit vom dem zum Zeitpunkt t0 übertragenen Wert der Referenzlichstärke im Falle einer übertragung in reinem, von Plättchen und weißen Blutkörperchen befreitem Plasma, wobei dies für jede Blutflüssigkeit gilt; des weiteren mit Verschlußvorrichtungen (25) für den Kreislauf zum Ablaß des Plasmas aus dem Behälter (5), die aktiviert werden und auf den Stutzen (10) einwirken, sobald das elektrische Signal (23) unter Bezugnahme auf die Referenzlichtstärke dem entsprechenden Wert der übertragenen Lichstärke entspricht.

5. Trennvorrichtung nach Anspruch 4, dadurch gekennzeichnet, daß sie mit Vorrichtungen (25) zum Verschluß des Stutzens (10) versehen ist, die hintereinandergeschaltet einen Speicher (26), einen Spannungsteiler (27), einen Spannungsvergleicher (28), einen Kippschaltung (29) und einen Stromkreis (30) umfassen, und mit Vorrichtungen (31) zur Aussendung eines dem Beginn des Plasmaflusses entsprechenden Signals sowie parallelgeschalteten Verzögerungselementen (32) versehen ist.

6. Auffassungsvorrichtung nach Anspruch 5, dadurch gekennzeichnet, daß sie mit einem Schaltkreis zur automatischen Erfassung (33) der unverwendbaren, ausgeschiedenen Blutplasmen versehen ist, der einen erweiterten, mit einem Vergleicher (34) und einer Kippschaltung (35) ausgestatteten Schaltkreis umfaßt.

**Claims**

1. A device for separating red blood cells from blood plasma composed of a flexible container (5), wich is transparent, particularly in artificial light, for blood which has undergone a centrifugal process beforehand, tubing (10) for the evacuation of the contents of the bag (5), a flat, vertical, fixed clamping jaw (2), and a flat, inclined, mobile clamping jaw (3) pivoting on a horizontal axe provided at the base of the fixed clamping jaw (2), the fixed clamping jaw (2) end the mobile clamping jaw (3) being joined together by elastic means drawing the mobile clamping jaw (3) towards the fixed clamping jaw (2), means (11) for supporting the container (5) in a fixed vertical position with regard to the vertical clamping jaw (2), means for emitting (6) and receiving (22) a beam of light passing through the walls (21, 21') of the container (5) and the blood,

and used for detecting the separation of white and red blood cells, characterised in that it comprises two arms (12 and 13), respectively bearing the emitting means (6) end the reception means (22) for the light beam, the said arms being kept parallel during plasma separation, and being temporarily separable in order to insert the container (5) or extract it with its means of support (11).

2. A separating device according to claim 1, characterised in that it comprises struts (14, 15) keeping the arms parallel during plasma separation.

3. A separating device according to claim 2, characterised in that the arms (12 and 13) are articulated with regard to each other around an axe (17), and in that it comprises a latch (16) capable of locking this articulation during plasma separation, and freeing it so that the container can be placed on the support means (11).

4. A separation device according to any one of claims 1 to 3, characterised in that it comprises means of transmitting en electric signal (23) depending on the intensity of the light emitted, and means for measuring the value of the light intensity transmitted at a moment $t^1$ with regard to the value of the light intensity transmitted at a moment $t^0$, taken as a reference, when the light is transmitted through the pure plasma devoid of platelets and white blood cells, and this is repeated for each different type of blood, means for closing (25) a flow circuit for the plasma outside the container (5), activated when the electric signal (23) corresponds to a given value of transmitted light intensity with regard to the light intensity taken as reference, and operating on the tubing (10).

5. A separation device according to claim 4, characterised in that it comprises means (25) of closing the tubing (10) comprising a memory (26), a voltage divider (27), a voltage comparator (28), a bistable trigger (29) and an energy circuit (30) placed in series, and means for transmitting (31) a signal corresponding to the start of plasma flow, and delaying means (32), placed parallel.

6. A detecting device according to claim 5, characterised in that it comprises an automatic detection circuit (33) for rejected plasma unfit for use, composed of a supplementary circuit comprising a voltage comparator (34) and a bistable trigger (35).

FIG. 6

FIG. 1

EP 0 175 618 B1

EP 0 175 618 B1

## FIG. 2

11

## FIG. 4

3

## FIG. 3

12

14

16

15

22

18

7

6

19

17

13

## FIG. 5

1

20

9

8

5

2

3

4

FIG. 7